(19) Europäisches Patentamt
European Patent Office
Office européen des brevets

(11) **EP 2 803 313 A1**

(12) **EUROPEAN PATENT APPLICATION**

(43) Date of publication:
**19.11.2014 Bulletin 2014/47**

(51) Int Cl.:
***A61B 1/00*** (2006.01)   ***A61B 1/06*** (2006.01)
***A61B 1/04*** (2006.01)

(21) Application number: **14167618.9**

(22) Date of filing: **09.05.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.05.2013 JP 2013102510**

(71) Applicant: **Fujifilm Corporation**
**Minato-ku**
**Tokyo (JP)**

(72) Inventor: **Shigeta, Norimasa**
**Kanagawa (JP)**

(74) Representative: **Klunker . Schmitt-Nilson . Hirsch**
**Patentanwälte**
**Destouchesstraße 68**
**80796 München (DE)**

(54) **Processor device, endoscope system, and operation method of endoscope system**

(57)     To display a brownish area distinguishably from residuum, a B image signal having a blue narrow-band signal corresponding to blue narrow-band light and a G image signal, out of a color image signal obtained by an endoscope (12), are inputted to a processor device (16). The B image signal is assigned as a B display image signal. The B display image signal and the G image signal are weighted independently, and the sum of the weighted B display image signal and the weighted G image signal is assigned as a G display image signal. The G image signal is assigned as an R display image signal. Based on the RGB display image signals, a special light image is displayed on a monitor (18). In the special light image, the residuum or residual fluid (80) is displayed in a yellowish color, and structure including blood vessels (82) and a lesion is visible translucently under the residuum (80).

FIG. 5B

EP 2 803 313 A1

**Description**

BACKGROUND OF THE INVENTION

1. Field of the Invention

The present invention relates to a processor device, an endoscope system, and an operation method of the endoscope system that perform special light observation such as blood vessel emphasized observation.

2. Description Related to the Prior Art

In a medical field, diagnosis and treatment using an endoscope system having a light source device, an endoscope, and a processor device are widely carried out. In endoscopy, not only normal observation to obtain an image of an observation object inside a living body that is likely to be visible by naked eyes under white light emitted from the light source device, but also special light observation (refer to US Patent Application Publication Nos. 2003/0176768, 2008/0294105, and 2008/0281154) is performed. The special light observation obtains an image in which blood vessels and the like in a superficial layer of mucus in the living body are emphasized by alternately applying blue narrow-band light and green narrow-band light, having a narrower band than the illumination light of the normal observation has, to the observation object.

In the above patent documents, for the purpose of emphasizing the superficial blood vessels and the like in display, a blue narrow-band signal obtained under irradiation with the blue narrow-band light is assigned as a blue display image signal and a green display image signal, and a green narrow-band signal obtained under irradiation with the green narrow-band light is assigned as a red display image signal. This facilitates improving contrast between the mucus and the superficial blood vessels and the like. However, such color assignment displays residuum, residual fluid, bile, intestinal juice, and the like in red, similarly to the color of blood, in an image. Therefore, a bleeding portion, which is suspected to be a lesion, is hard to distinguish from the residuum and the like in the image. This easily causes misdiagnosis.

On the other hand, according to US Patent Application Publication No. 2009/0036741, the blue narrow-band signal is assigned as the green display image signal, and the green narrow-band signal is assigned as the blue display image signal and the red display image signal, so that the residuum and the like are displayed in magenta. This allows distinction of the residuum and the like from the bleeding portion.

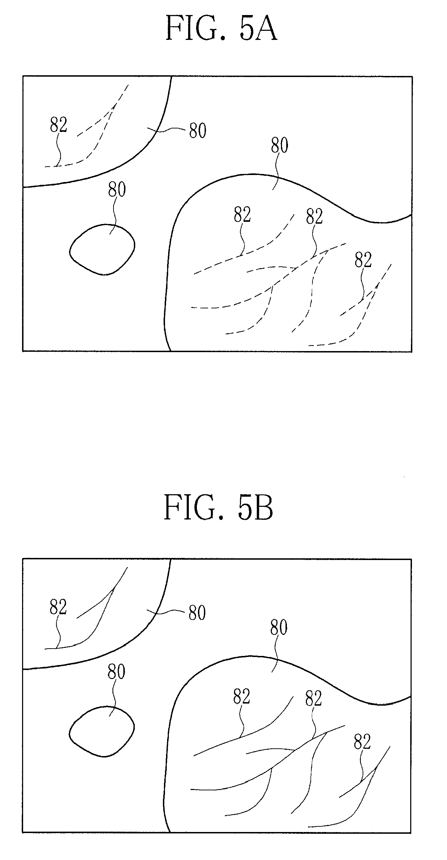

However, displaying the residuum and the like in magenta makes various types of structure under the residuum, such as the blood vessels, hard to see (refer to Fig. 5A). This may cause failure to find out a lesion. Furthermore, in addition to the residuum and the like, a part of the superficial blood vessels, a brownish area, and the like are sometimes displayed in magenta. In some cases, the brownish area cannot be clearly distinguished from the residuum and the like.

SUMMARY OF THE INVENTION

An obj ect of the present invention is to provide a processor device, an endoscope system, and an operation method of the endoscope system that make various types of structure, including blood vessels and the like, visible translucently under residuum and the like and display a brownish area distinguishably from the residuum and the like.

To achieve the above and other objects, a processor device according to the present invention applies image processing to a color image signal obtained by an endoscope to convert the color image signal into blue, green, and red display signals, and supplies the blue, green, and red display signals to a display means for displaying an image of an observation object. The processor device includes an image signal input means, a color conversion means, and a display signal producing means. The image signal input means captures, out of the color image signal, a blue image signal containing a blue narrow-band signal corresponding to a wavelength band in a blue region and a green image signal corresponding to a wavelength band in a green region. The color conversion means assigns the blue image signal as a blue display image signal, and assigns a sum of the weighted green image signal and the weighted blue image signal as a green display image signal, and assigns the green image signal as a red display image signal. The display signal producing means converts the blue display image signal, the green display image signal, and the red display image signal into the blue, green, and red display signals, respectively.

The color conversion means preferably includes a color feature value calculator and a weighting coefficient calculator. The color feature value calculator calculates a color feature value of the observation object based on the color image signal. The weighting coefficient calculator calculates, in accordance with the color feature value, a first weighting coefficient for weighting the green image signal and a second weighting coefficient for weighting the blue image signal.

The color feature value preferably increases with getting near a color of a first object including at least one of residuum, residual fluid, bile, and intestinal juice present on the observation object, while the color feature value preferably decreases with getting near a color of a second object including a brownish area. With increase in the color feature value, the first weighting coefficient for weighting the green image signal is preferably increased, and the second weighting coefficient for weighting the blue image signal is preferably decreased.

The sum of the first weighting coefficient and the second weighting coefficient is preferably "1". In a case where the color feature value is equal to or less than a first threshold value, the first weighting coefficient may be set at "0", and the second weighting coefficient may be set at "1". In a case where the color feature value is equal to or more than a second threshold value, being larger than the first threshold value, the first weighting coefficient may be set at "1", and the second weighting coefficient may be set at "0". In a case where the color feature value is between the first threshold value and the second threshold value, the first weighting coefficient may be increased and the second weighting coefficient may be decreased with increase in the color feature value.

The color feature value is preferably calculated based on first color information that is obtained by an operation of the blue image signal and the green image signal.

The image signal input means may capture a red image signal corresponding to a wavelength band in a red region, in addition to the blue image signal and the green image signal. The color feature value may be calculated based on not only the first color information but also second color information that is obtained by an operation of the green image signal and the red image signal.

It is preferable that the color feature value is the sum of the weighted first color information and the weighted second color information, and the first color information is more heavily weighted than the second color information.

A first object including at least one of residuum, residual fluid, bile, and intestinal juice present on the observation object is displayed in a yellowish color on the display means. Structure under the first object is displayed translucently on the display means.

An endoscope system according to the present invention includes an endoscope for taking an image of an observation object and outputting a color image signal, a processor device, and a display means for displaying the image of the observation object based on blue, green, and red display signals. The processor device includes an image signal input means, a color conversion means, and a display signal producing means. The image signal input means captures, out of the color image signal, a blue image signal containing a blue narrow-band signal corresponding to a wavelength band in a blue region and a green image signal corresponding to a wavelength band in a green region. The color conversion means assigns the blue image signal as a blue display image signal, and assigns the sum of the weighted green image signal and the weighted blue image signal as a green display image signal, and assigns the green image signal as a red display image signal. The display signal producing means converts the blue display image signal, the green display image signal, and the red display image signal into the blue, green, and red display signals, respectively.

The endoscope preferably includes a first lighting means for applying illumination light containing blue narrow-band light to the observation object, and a color image pickup device for taking the image of the observation object and outputting the blue image signal and the green image signal. Otherwise, the endoscope preferably includes a second lighting means for alternately applying blue narrow-band light and green narrow-band light to the observation object, and a monochrome image pickup device. The monochrome image pickup device takes the image of the observation object, and outputs the blue image signal containing the blue narrow-band signal corresponding to the blue narrow-band light, and outputs the green image signal containing a green narrow-band signal corresponding to the green narrow-band light. Otherwise, the endoscope preferably includes a third lighting means for applying broad-band light in a wavelength band extending from the blue region to the red region to the observation object, a color image pickup device for taking the image of the observation object and outputting the color image signal, and a spectral operation unit for producing the blue image signal and the green image signal by a spectral operation of the color image signal.

An operation method of an endoscope system according to the present invention includes the steps of actuating an image signal input means and capturing, out of a color image signal obtained by an endoscope, a blue image signal containing a blue narrow-band signal corresponding to a wavelength band in a blue region and a green image signal corresponding to a wavelength band in a green region; actuating a color conversion means and assigning the blue image signal as a blue display image signal, and assigning the sum of the weighted green image signal and the weighted blue image signal as a green display image signal, and assigning the green image signal as a red display image signal; actuating a display signal producing means and converting the blue display image signal, the green display image signal, and the red display image signal into blue, green, and red display signals, respectively; and displaying an image of an observation object on a display means based on the blue, green, and red display signals.

According to the present invention, it is possible to make various types of structure such as blood vessels visible translucently under the residuum and the like, and display a brownish area distinguishably from the residuum and the like.

BRIEF DESCRIPTION OF DRAWINGS

For more complete understanding of the present invention, and the advantage thereof, reference is now made to the subsequent descriptions taken in conjunction with the accompanying drawings, in which:

Fig. 1 is a schematic view of an endoscope system;
Fig. 2 is a block diagram showing the structure of the endoscope system according to a first embodiment;
Fig. 3A is a graph showing an emission spectrum of white light;
Fig. 3B is a graph showing an emission spectrum of special light;
Fig. 4A is a graph showing spectral transmittance of a B filter, a G filter, and an R filter provided in a primary color image sensor;
Fig. 4B is a graph showing spectral transmittance of a Y (yellow) filter, a M (magenta) filter, a C (cyan) filter, and a G (green) filter provided in a complementary color image sensor;
Fig. 5A is a drawing of a special light image in the case of a weighting coefficient w1 of "0";
Fig. 5B is a drawing of a special light image in a case where the weighting coefficient w1 is more than "0" and less than "1";
Fig. 6 is a graph showing the color distribution of residuum or residual fluid and a brownish area in two-dimensional color space;
Fig. 7 is a graph showing the input-output relation of 1DLUT;
Fig. 8 is a flowchart showing the operation of the present invention;
Fig. 9 is a block diagram showing the structure of an endoscope system that is provided with a phosphor in a light source device;
Fig. 10 is a block diagram of an endoscope system according to a second embodiment;
Fig. 11 is a plan view of a rotary filter;
Fig. 12 is a graph showing spectral transmittance of a Bn filter and a Gn filter; and
Fig. 13 is a block diagram of a special light image processor having a spectral operation unit.

DESCRIPTION OF THE PREFERRED EMBODIMENTS

(First Embodiment)

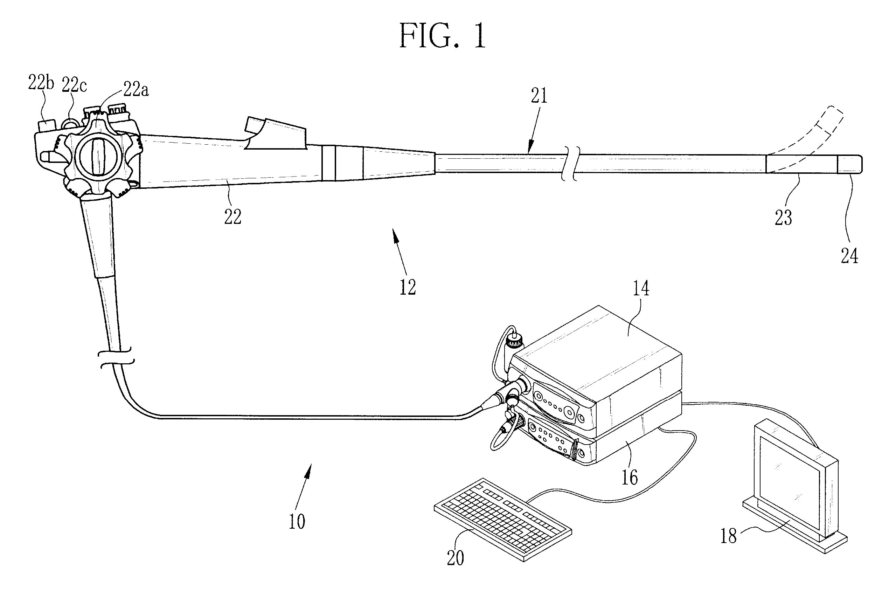

As shown in Fig. 1, an endoscope system 10 according to a first embodiment has an endoscope 12, a light source device 14, a processor device 16, a monitor 18, and a console 20. The endoscope 12 is optically connected to the light source device 14, and electrically connected to the processor device 16. The endoscope 12 has an insert section 21 to be introduced into a body cavity, a control handle unit 22 provided at a proximal end of the insert section 21, and a steering assembly 23 and a head assembly 24 provided at a distal end of the insert section 21. The steering assembly 23 bends by operating an angle knob 22a on the control handle unit 22. This bending operation directs the head assembly 24 to a desired direction.

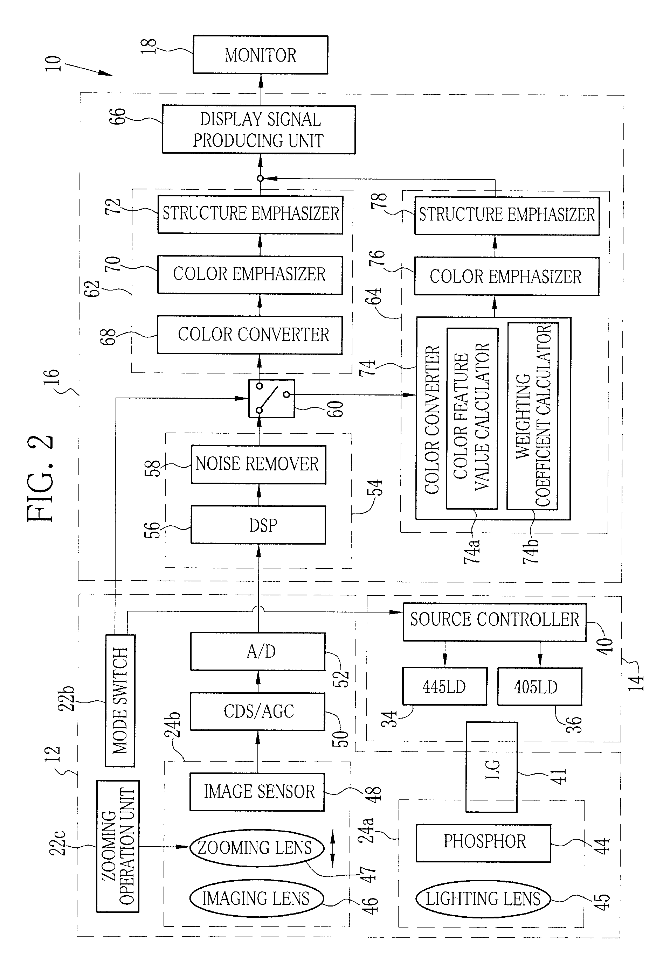

The control handle unit 22 is provided with a mode switch 22b and a zooming operation unit 22c, in addition to the angle knob 22a. The mode switch 22b is used for switching operation between two modes, i.e. a normal observation mode and a special observation mode. In the normal observation mode, a normal light image captured under white light is displayed on the monitor 18. In the special observation mode, a special light image captured using bluish special light is displayed on the monitor 18. The zooming operation unit 22c is used for zooming operation by which an observation object is scaled up by operating a zooming lens 47 (see Fig. 2) of the endoscope 12.

The processor device 16 is electrically connected to the monitor 18 and the console 20. The monitor 18 displays image information and the like. The console 20 functions as a UI (user interface) that receives the input of functional settings and the like. Note that, an external storage (not shown) for storing the image information and the like may be connected to the processor device 16.

As shown in Fig. 2, the light source device 14 is provided with a blue laser source (445LD) 34 that emits a blue laser beam having a center wavelength of 445 nm, and a violet laser source (405LD) 36 that emits a violet laser beam having a center wavelength of 405 nm, as light sources. A source controller 40 controls light emission from a semiconductor light emitting element of each laser source 34, 36 independently, so that the light amount ratio between emission light from the blue laser source 34 and emission light from the violet laser source 36 is flexibly changeable. In the normal observation mode, the source controller 40 actuates only the blue laser source 34. In the special observation mode, the source controller 40 actuates both of the blue laser source 34 and the violet laser source 36, and makes the emission rate of the violet laser beam higher than the emission rate of the blue laser beam.

Note that, the full width at half maximum of the blue laser beam or the violet laser beam is preferably set at the order of ±10 nm. The violet laser source 36 may be actuated in the normal observation mode too. In this case, however, the emission intensity of the violet laser beam is preferably set low. As the blue laser source 34 and the violet laser source 36, a broad-area type InGaN laser diode, InGaNAs laser diode, or GaNAs laser diode is available. Also, an illuminant such as a light emitting diode may be used as the light sources described above.

The laser beam emitted from each of the laser sources 34 and 36 enters a light guide (LG) 41 through optical members (none of them is shown) including a condenser lens, an optical fiber, a multiplexer, and the like. The light guide 41 is contained in a universal cord (not shown) for connecting the light source device 14 and the endoscope 12. The blue laser beam having the center wavelength of 445 nm or the violet laser beam having the center wavelength of 405 nm is transmitted to the head assembly 24 of the endoscope 12 through the light guide 41. Note that, a multi-mode fiber is available as the light guide 41. By way of example, a slender fiber cable is available that has a core diameter of 105 $\mu$m, a clad diameter of 125 $\mu$m, and a diameter $\phi$ of 0.3 to 0.5 mm including a protective layer being a sheath.

The head assembly 24 of the endoscope 12 has a lighting optical system 24a and an imaging optical system 24b. The lighting optical system 24a is provided with a lighting lens 45 and a phosphor 44 upon which the blue laser beam having the center wavelength of 445 nm or the violet laser beam having the center wavelength of 405 nm is incident from the light guide 41. By the incidence of the blue laser beam on the phosphor 44, the phosphor 44 emits fluorescence. A part of the blue laser beam passes through the phosphor 44, without being absorbed therein. The violet laser beam passes through the phosphor 44 without exciting the phosphor 44. Light ejected from the phosphor 44 is applied to the observation object inside the body cavity through the lighting lens 45.

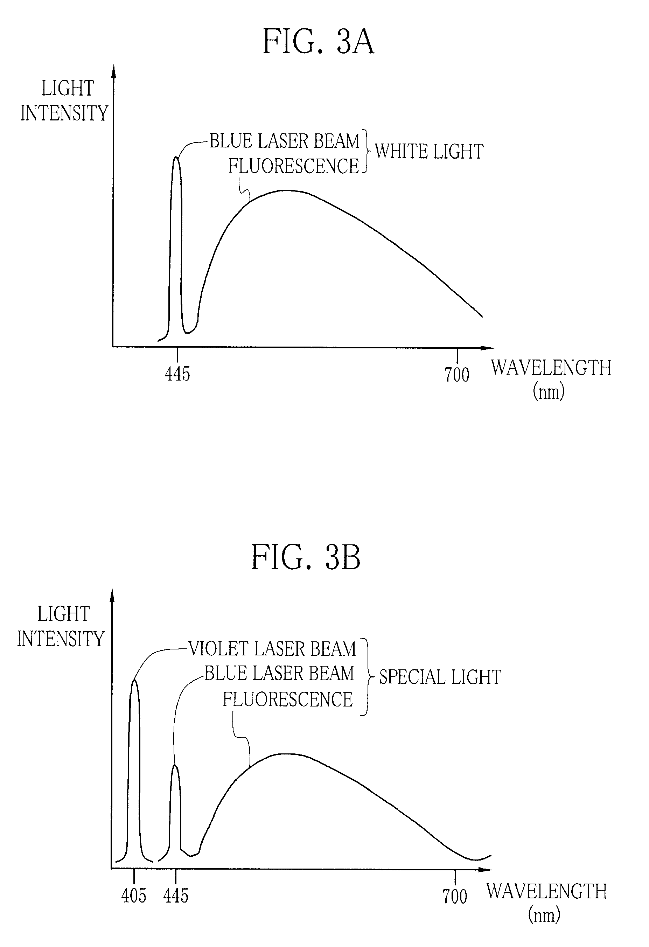

In the normal observation mode, only the blue laser beam is incident upon the phosphor 44. Thus, as shown in Fig. 3A, white light, being a combination of the blue laser beam and the fluorescence emitted from the phosphor 44 excited by the blue laser beam, is applied to the observation object. In the special observation mode, on the other hand, both of the violet laser beam and the blue laser beam are incident upon the phosphor 44. Thus, as shown in Fig. 3B, special light, being a combination of the violet laser beam, the blue laser beam, and the fluorescence emitted from the phosphor 44 excited by the blue laser beam, is applied to the observation object. In the special observation mode, the emission rate of the violet laser beam is set higher than the emission rate of the blue laser beam. Therefore, the special light contains a blue component at a high ratio, and the wavelength band of the special light extends almost over the entire visible region.

Note that, the phosphor 44 preferably contains a plurality of types of fluorescent materials (for example, a YAG phosphor or a phosphor such as BAM ($BaMgAl_{10}O_{17}$)) that absorb a part of the blue laser beam and emit green to yellow pumped light. The use of the semiconductor light emitting element as a pumping source of the phosphor 44, as being described in this embodiment, allows obtainment of the white light of high intensity at high luminous efficiency. Therefore, it is possible to easily adjust the intensity of the white light and restrain variation in color temperature and chromaticity of the white light.

As shown in Fig. 2, the imaging optical system 24b of the endoscope 12 has an imaging lens 46, a zooming lens 47, and an image sensor (image pickup device) 48. Light reflected from the observation object is incident upon the image sensor 48 through the imaging lens 46 and the zooming lens 47. Thus, a reflected image of the observation object is formed on the image sensor 48. The zooming lens 47 shifts between a telephoto position and a wide-angle position by operation of a zooming operation unit 22c. The shift of the zooming lens 47 to the telephoto position scales up the reflected image of the observation object, while the shift of the zooming lens 47 to the wide-angle position scales down the reflected image of the observation object.

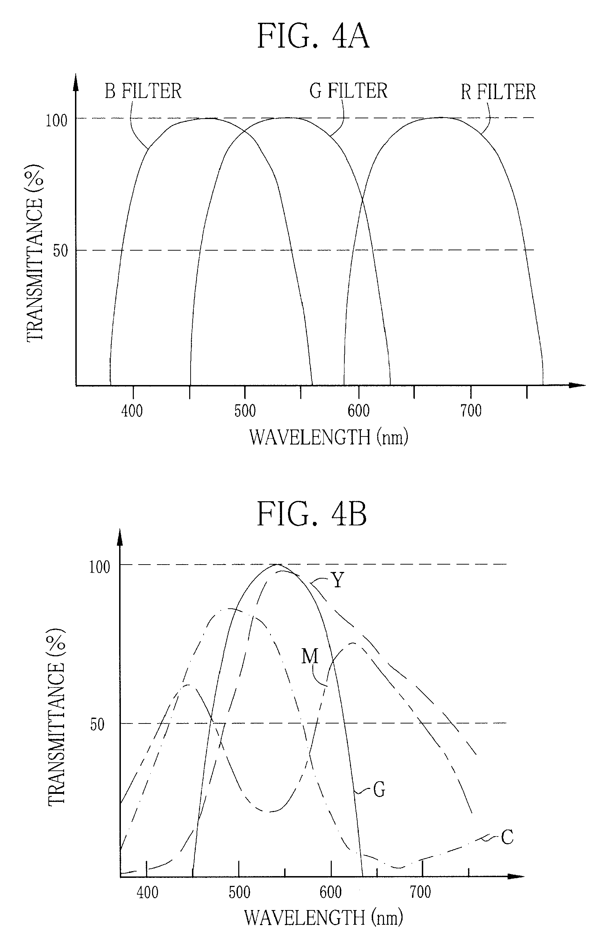

The image sensor 48, which has a color mosaic filter, captures the reflected image of the observation object and outputs color image signals. Note that, the image sensor 48 is preferably a CCD (charge coupled device) image sensor, a CMOS (complementary metal-oxide semiconductor) image sensor, or the like. The image sensor used in this invention is a so-called RGB image sensor, which has an RGB filter on its imaging surface to obtain image signals of three colors of R (red), G (green), and B (blue). As shown in Fig. 4A, the B filter of the RGB image sensor passes light of 380 to 570 nm. The G filter passes light of 450 to 630 nm. The R filter passes light of 580 to 770 nm.

Note that, the image sensor 48 may be a so-called complementary color image sensor having a complementary color filter of C (cyan), M (magenta), Y (yellow), and G (green), which has spectral transmittance as shown in Fig. 4B, instead of the RGB image sensor. In the case of the complementary color image sensor, RGB three-color image signals can be obtained from CMYG four-color image signals by color conversion. In this case, a color conversion means that performs the color conversion from the CMYG four-color image signals to the RGB three-color image signals is necessarily provided in any one of the endoscope 12, the light source device 14, and the processor device 16.

The image signals outputted from the image sensor 48 are transmitted to a CDS/AGC circuit 50. The CDS/AGC circuit 50 applies correlation double sampling (CDS) and automatic gain control (AGC) to the image signals, being analog signals. The image signals processed by the CDS/AGC circuit 50 are converted into digital image signals by an A/D converter 52. The converted digital image signals are inputted to the processor device 16.

The processor device 16 is provided with a receiver 54, an image processing switching unit 60, a normal light image processor 62, a special light image processor 64, and a display signal producing unit 66. The receiver 54 receives the RGB digital image signals from the endoscope 12. The R image signals correspond to signals outputted from R pixels (pixels having the R filters) of the image sensor 48. The G image signals correspond to signals outputted from G pixels (pixels having the G filters) of the image sensor 48. The B image signals correspond to signals outputted from B pixels (pixels having the B filters) of the image sensor 48. The receiver 54 includes a DSP (digital signal processor) 56 and a noise remover 58. The DSP 56 applies gamma correction and color correction processing to the RGB image signals. The noise remover 58 applies noise removal processing (for example, a method of moving averages, a median filter method, or the like) to the RGB image signals after being subjected to the gamma correction and the like in the DSP 56, to remove noise from the RGB image signals. After the noise removal, the RGB image signals are transmitted to the image processing switching unit 60.

Provided that the endoscope system 10 is put in the normal observation mode by the operation of the mode switch 22b, the image processing switching unit 60 transmits the RGB image signals to the normal light image processor 62. In the case of the special observation mode, the image processing switching unit 60 transmits the RGB image signals to the special light image processor 64. Out of the RGB image signals to be transmitted to the special light image processor 64, the B image signal contains a blue narrow-band signal that corresponds to the blue laser beam and the violet laser beam, out of the special light, that are in wavelength bands having a high light absorption coefficient of hemoglobin.

The normal light image processor 62 has a color converter 68, a color emphasizer 70, and a structure emphasizer 72. The normal light image processor 62 produces a normal light image, which represents the inside of the body cavity in normal color of a living body. The color converter 68 makes a matrix conversion of the RGB three-channel digital image signals (denoted as Ri, Gi, and Bi) based on the following expression (1), and outputs RGB display image signals ($R_0$, Go, and $B_0$). The RGB display image signals are further subjected to gradation conversion processing and the like, and made into color-converted RGB image signals. Note that, in the expression (1), k1, k2, and k3 represent arbitrary constants.

$$\begin{pmatrix} Ro \\ Go \\ Bo \end{pmatrix} = \begin{pmatrix} k1 & 0 & 0 \\ 0 & k2 & 0 \\ 0 & 0 & k3 \end{pmatrix} \times \begin{pmatrix} Ri \\ Gi \\ Bi \end{pmatrix} \cdots (1)$$

The color emphasizer 70 applies various types of color emphasizing processing to the color-converted RGB image signals. The structure emphasizer 72 applies structure emphasizing processing including sharpness, outline emphasis, and the like to the color-emphasized RGB image signals. The RGB image signals whose structure is emphasized by the structure emphasizer 72 are inputted as the normal light image from the normal light image processor 62 to the display signal producing unit 66.

The special light image processor 64 has a color converter 74, a color emphasizer 76, and a structure emphasizer 78. The special light image processor 64 produces a special light image in which superficial blood vessels and the like are emphasized and residuum, residual fluid, bile, or intestinal juice (hereinafter simply called "residuum or residual fluid") is displayed in a yellowish color in distinction from a bleeding portion. The color converter 74 makes a matrix conversion of the RBG three-channel digital image signals (denoted as Ri, Gi, and Bi) based on the following expression (2), and outputs RBG display image signals (denoted as $R_0$, Go, and $B_0$). The RGB display image signals are further subjected to the gradation conversion processing and the like, and made into color-converted RGB image signals.

$$\begin{pmatrix} Ro \\ Go \\ Bo \end{pmatrix} = \begin{pmatrix} 0 & 1 & 0 \\ 0 & w1 & w2 \\ 0 & 0 & 1 \end{pmatrix} \times \begin{pmatrix} Ri \\ Gi \\ Bi \end{pmatrix} \cdots (2)$$

In the expression (2), "w1" represents a weighting coefficient by which the G image signal is multiplied. "w2" is a weighting coefficient by which the B image signal is multiplied. A relation of w2=1-w1 holds true. If w1 is "0" and w2 is "1", ($R_0$, Go, $B_0$) = (Gi, Bi, Bi). In this case, the residuum or residual fluid is hardly contained in the B image signal (Bi; the pixel value of the B image signal is almost "0"), but much contained in the G image signal (Gi). Thus, the residuum or residual fluid is displayed in bright red on the monitor 18. Accordingly, the residuum or residual fluid is in the same color as the bleeding portion. Structure information, including information about the blood vessels and the like under the residuum or residual fluid, is hardly contained in the B image signal (Bi), but much contained in the G image signal (Gi). Thus, if the B image signal (Bi) is assigned as the G display image signal ($G_0$), as shown in Fig. 5A, various types of structure 82 including the blood vessels, a lesion, and the like under the residuum or residual fluid 80 are not displayed in original colors, and hence probably invisible by being obstructed by the residuum or residual fluid 80.

Therefore, increasing w1 between "0" and "1" and adding the G image signal (Gi) to the G display image signal ($G_0$) make it possible to display the special light image in almost the same colors as the normal light image. Accordingly, the residuum or residual fluid is displayed in a yellowish color in the special light image. This allows making distinction between the residuum or residual fluid and the bleeding portion. Furthermore, as shown in Fig. 5B, the various types of structure 82 including the blood vessels, the lesion, and the like under the residuum or residual fluid 80 are displayed in their original colors, and the structure 82 becomes visible without being obstructed by the residuum or residual fluid. Therefore, even if the residuum or residual fluid is present, it is possible to more visibly display information about the blood vessels, the lesion, and the like under the residuum or residual fluid. Note that, the color of the residuum or residual fluid approaches "yellow", being its original color, with increase in w1. Thus, w1 is a coefficient that indicates reality of the residuum or residual fluid.

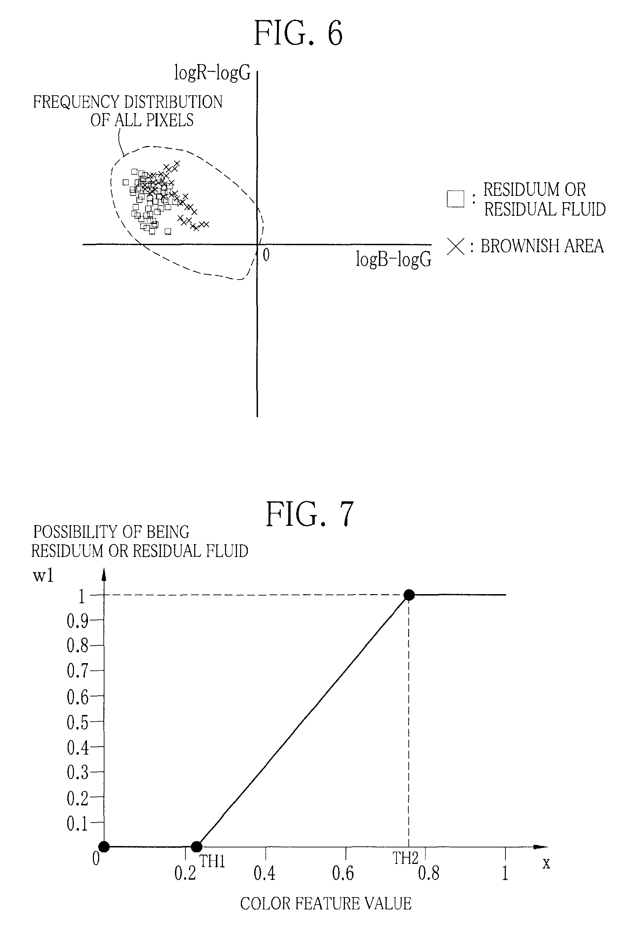

In two-dimensional space in which a horizontal axis represents " log B-log G" and a vertical axis represents "log R-log G", as shown in Fig. 6, values (denoted by □) of pixels having the residuum or residual fluid approximate to values (denoted by x) of pixels of a brownish area, being suspected to be a lesion. In other words, the color of the residuum or residual fluid resembles the color of the brownish area. Accordingly, in the case of setting w1 and w2 at constants between "0" and "1" and making the matrix conversion based on the above expression (2), the brownish area is possibly displayed in a yellowish color, being the same color as the residuum or residual fluid. In this case, the brownish area is hard to find out and hence the lesion is missed easily. Thus, the color converter 74 displays the residuum or residual fluid in the "yellowish" color, being its original color, while setting the weighting coefficients w1 and w2 in accordance with a color feature value x of each pixel, for the purpose of displaying the brownish area in a color other than "yellow". Note that, the horizontal axis "log B-log G" of Fig. 6 is a value varying in accordance with the depth of the blood vessel (the position of the blood vessel in a depth direction in mucosal tissue). The vertical axis "log R-log G" is a value varying in accordance with blood volume (the quantity of hemoglobin in blood).

A color feature value calculator 74a calculates the color feature value x (a numerical value between "0" and "1") by the following expression (3) based on the RGB image signals (Ri, Gi, and Bi).

$$x = a \times \left| \log Bi - \log Gi \right| + b \times \left| \log Ri - \log Gi \right| \cdots (3)$$

In the expression (3), "Ri", "Gi", and "Bi" represent the R image signal, the G image signal, and the B image signal, respectively. "a" and "b" are constants determined in advance from the color distribution of the residuum or residual fluid and the brownish area. As shown in Fig. 6, since the difference in "log B-log G" between the residuum or residual fluid and the brownish area is larger than the difference in "log R-log G" therebetween, "a" is set at 0.8 and "b" is set at 0.2 in this embodiment. Considering that "log B-log G" decreases, or equivalently, "|log B-log G|" increases with nearing the color of the residuum or residual fluid, the color feature value x decreases with getting near the color of the brownish area, and the color feature value x increases with getting near the color of the residuum or the residual fluid. Note that, the color feature value x is represented by using the difference between the G image signal and the B image signal and the difference between the G image signal and the R image signal, but may be represented by a value obtained by another calculation.

The weighting coefficient calculator 74b calculates the weighting coefficients w1 and w2 based on the color feature value x. The weighting coefficient w1 is calculated using a 1DLUT(x), which stores the correlation between the color feature value x and the weighting coefficient w1. The 1DLUT (one-dimensional lookup table) has nonlinear input/output relation, as shown in Fig. 7. On the other hand, the weighting coefficient w2 is obtained by subtracting the weighting coefficient w1 from "1". Thus, in a case where the color feature value x is equal to or less than a first threshold value TH1, the weighting coefficient w1 is "0", while the weighting coefficient w2 is "1". In a case where the color feature value x is more than the first threshold value TH1, the weighting coefficient w1 increases and the weighting coefficient w2 decreases with increase in the color feature value x. In a case where the color feature value x is equal to or more than a second threshold value TH2, the weighting coefficient w1 is "1", while the weighting coefficient w2 is "0".

By varying the weighting coefficients w1 and w2 in accordance with the color feature value x, as described above, the matrix conversion based on the above expression (2) is turned to be not a simple matrix conversion, but a nonlinear matrix conversion that makes the residuum or residual fluid into "yellow" , being its original color, with maintaining some degree of contrast of the brownish area.

The color emphasizer 76 applies the various types of color emphasizing processing to the color-converted RGB image signals. The structure emphasizer 78 applies the structure emphasizing processing such as the sharpness and the outline emphasis to the color-emphasized RGB image signals. The RGB image signals after being subjected to the structure emphasizing processing by the structure emphasizer 78 are inputted as the special light image from the special light image processor 64 to the display signal producing unit 66.

The display signal producing unit 66 converts the normal light image or the special light image inputted from the normal light image processor 62 or the special light image processor 64 into BGR display signals (video signals), which are displayable on the monitor 18. Based on the converted display signals, the monitor 18 displays the normal light image or the special light image.

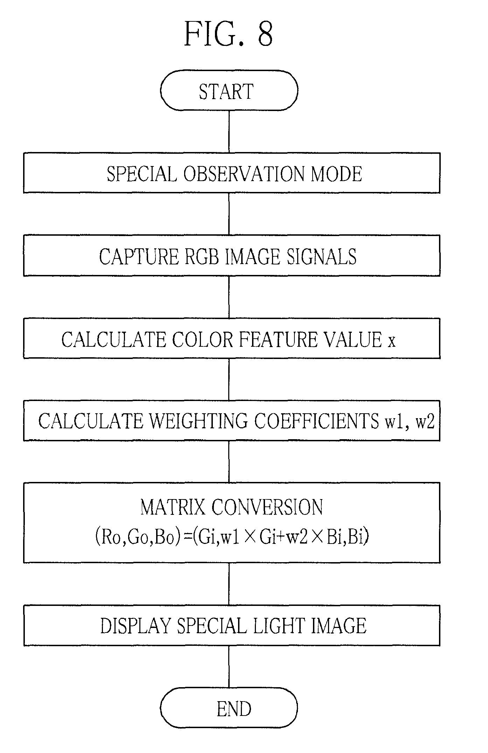

Next, the operation of the present invention will be described with referring to a flowchart of Fig. 8. First, the endoscope system 10 is put into the special observation mode by the operation of the mode switch 22b. Thus, the special light is applied to the observation object inside the body cavity. The image sensor 48 captures an image of the observation object under irradiation with the special light. Accordingly, the RGB image signals are obtained. The special light image processor 64 applies various types of image processing to the RGB image signals.

In the special light image processor 64, the color feature value calculator 74a calculates the color feature value x based on the RGB image signals. The color feature value x becomes small at pixels corresponding to the brownish area. On the contrary, the color feature value x becomes large at pixels corresponding to the residuum or residual fluid. The weighting coefficient calculator 74b calculates the weighting coefficients w1 and w2 from the color feature value x. Provided that the color feature value x is a constant value or more, the weighting coefficient w1 is more than "0". Then, the RGB image signals (Ri, Gi, Bi) are converted into the RGB display image signals (denoted as $R_0$, Go, $B_0$) by the matrix conversion based on the expression (2). Based on the display image signals, the special light image is displayed on the monitor 18.

In the special light image, the residuum or residual fluid is displayed in the "yellowish" color, so that the residuum or residual fluid is distinguishable from the bleeding portion. The various types of structure 82 such as the blood vessels and the lesion under the residuum or residual fluid 80 are displayed translucently in their original colors (see Fig. 5B). Moreover, by applying the nonlinear matrix conversion in accordance with the color feature value x, the brownish area is displayed in a color different from "yellow", though the residuum or residual fluid is displayed in the "yellowish" color, being its original color. This makes it possible to distinguish the brownish area from the residuum or residual fluid.

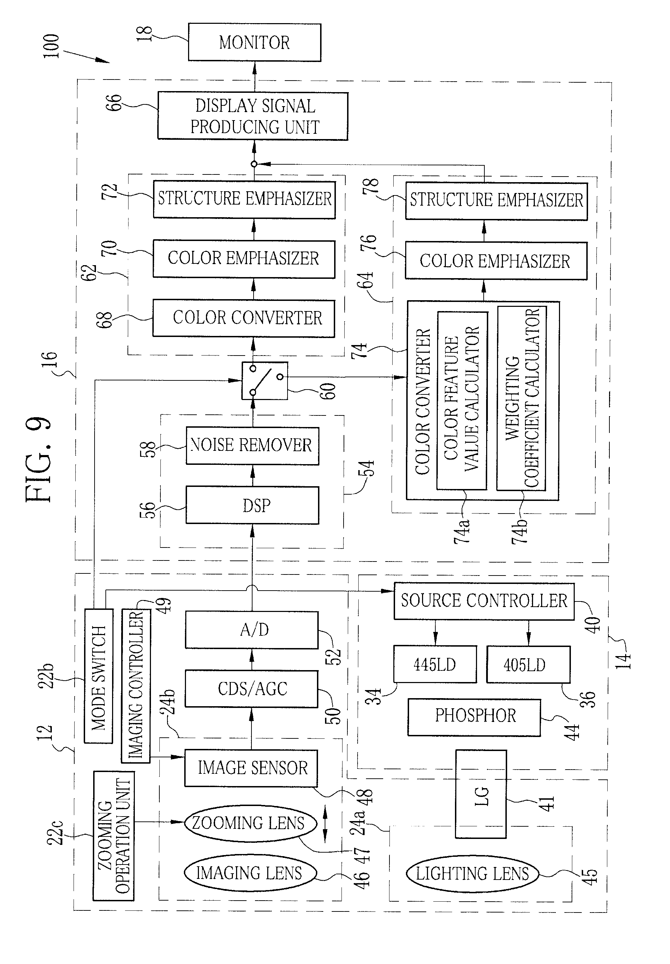

In the above first embodiment, the phosphor 44 is provided in the head assembly 24 of the endoscope 12. However, the phosphor 44 may be provided in the light source device 14 instead, as shown in an endoscope system 100 of Fig. 9. In this case, the phosphor 44 is provided between the blue laser source (445LD) 34 and the light guide 41 and between the violet laser source (405LD) 36 and the light guide 41. In the normal observation mode, the blue laser beam is applied from the blue laser source 34 to the phosphor 44, so that the white light is ejected from the phosphor 44. In the special observation mode, on the other hand, the blue laser beam and the violet laser beam are applied from the blue laser source 34 and the violet laser source 36, respectively, to the phosphor 44, so that the special light is ejected from the phosphor 44. The white light or the special light is applied to the observation object through the light guide 41. The other components of the endoscope system 100 are the same as those of the endoscope system 10.

(Second Embodiment)

The above first embodiment adopts a simultaneous method by which a plurality of image signals required in each observation mode is simultaneously obtained using a color image sensor. The present invention may adopt a frame sequential method by which a plurality of image signals required in each observation mode is sequentially obtained using a monochrome image sensor, instead.

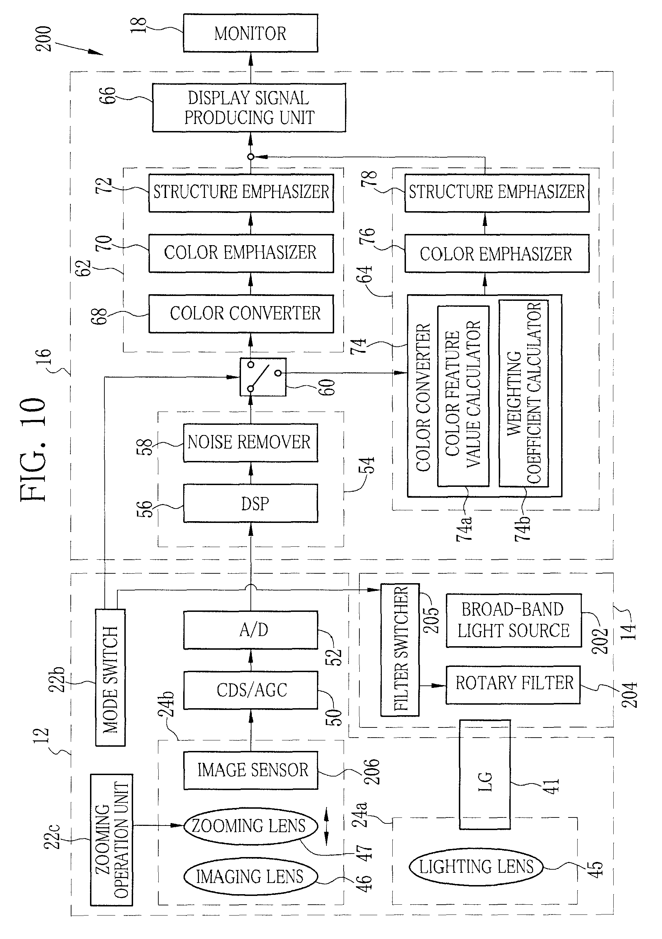

According to a frame sequential method type endoscope system 200, as shown in Fig. 10, the light source device 14 is provided with a broad-band light source 202, a rotary filter 204, and a filter switcher 205, instead of the blue laser source 34, the violet laser source 36, and the source controller 40. The lighting optical system 24a of the endoscope 12 has no phosphor 44. The imaging optical system 24b is provided with a monochrome image sensor 206 having no color filter, instead of the color image sensor 48. The other components of the endoscope system 200 are the same as those of the endoscope system 10 according to the first embodiment.

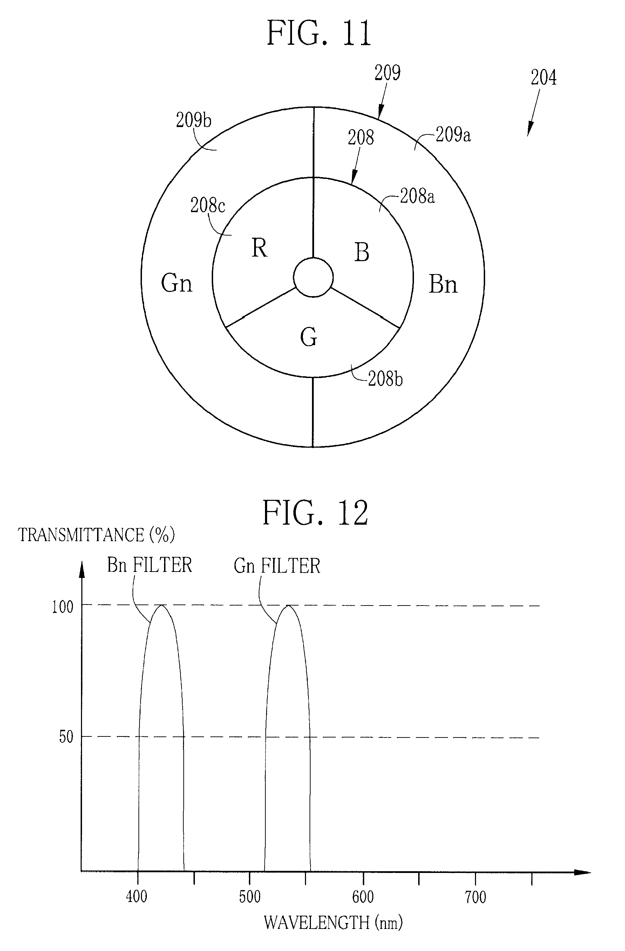

The broad-band light source 202 is a xenon lamp, a white LED, or the like, and emits white light in a wavelength band extending from the blue region to the red region. The rotary filter 204 includes an inner filter 208 for use in the normal observation mode and an outer filter 209 for use in the special observation mode (see Fig. 11). The filter switcher 205 shifts the rotary filter 204 in its radial direction. In a case where the endoscope system 200 is put in the normal observation mode by operation of the mode switch 22b, the inner filter 208 of the rotary filter 204 is inserted in an optical path of the white light. In the special observation mode, the outer filter 209 of the rotary filter 204 is inserted in the optical path of the white light.

As shown in Fig. 11, the inner filter 208 for use in the normal observation mode has a B filter 208a for passing blue light out of the white light, a G filter 208b for passing green light out of the white light, and an R filter 208c for passing red light out of the white light along its circumferential direction. In the normal observation mode, the blue light, the green light, and the red light are applied in turn to the observation object by rotation of the rotary filter 204. The transmittance of the BGR filters 208a to 208c is the same as that of the BGR filters of the image sensor 48 according to the first embodiment (see Fig. 4A).

The outer filter 209 for use in the special observation mode includes a Bn filter 209a for passing blue narrow-band light having a center wavelength of 415 nm out of the white light, and a Gn filter 209b for passing green narrow-band light having a center wavelength of 540 nm out of the white light, provided in its circumferential direction. Thus, in the special observation mode, the blue narrow-band light and the green narrow-band light are alternately applied to the observation object by rotation of the rotary filter 204. Note that, as shown in Fig. 12, the transmittance of the Bn filter 209a is high at 400 to 440 nm. The transmittance of the Gn filter 209b is high at 520 to 560 nm.

According to the endoscope system 200 of the frame sequential method, in the normal observation mode, the monochrome image sensor 206 captures images of the observation object whenever the blue light, the green light, and the red light are applied to the observation object. Thus, RGB three-color image signals are obtained. Then, the normal light image is produced from the RGB image signals in the same way as the above first embodiment.

In the special observation mode, on the other hand, the monochrome image sensor 206 captures images of the observation object whenever the blue narrow-band light and the green narrow-band light are applied to the observation object. Thus, a Bn image signal and a Gn image signal are obtained. The special light image processor 64 makes a matrix conversion of the Bn image signal and the Gn image signal (denoted as Bn and Gn) based on the following expression (4), to output RGB display image signals (denoted as $R_0$, Go, $B_0$). Then, the special light image is produced from the RGB display image signals in the same way as the above first embodiment.

$$\begin{pmatrix} Ro \\ Go \\ Bo \end{pmatrix} = \begin{pmatrix} 1 & 0 \\ w1 & w2 \\ 0 & 1 \end{pmatrix} \times \begin{pmatrix} Gn \\ Bn \end{pmatrix} \cdots (4)$$

Note that, in the second embodiment, the color feature value x, which is used in calculation of the weighting coefficients w1 and w2, is calculated by the following expression (5), based on the Bn image signal and the Gn image signal (denoted as Bn and Gn).

$$x = \left| \log Bn - \log Gn \right| \cdots (5)$$

(Third Embodiment)

In a third embodiment, the Bn image signal having information at a wavelength of 415 nm and the Gn image signal having information at a wavelength of 540 nm are produced by applying spectral operation to a broad-band image such as a white light image. The special light image is produced from the Bn image signal and the Gn image signal.

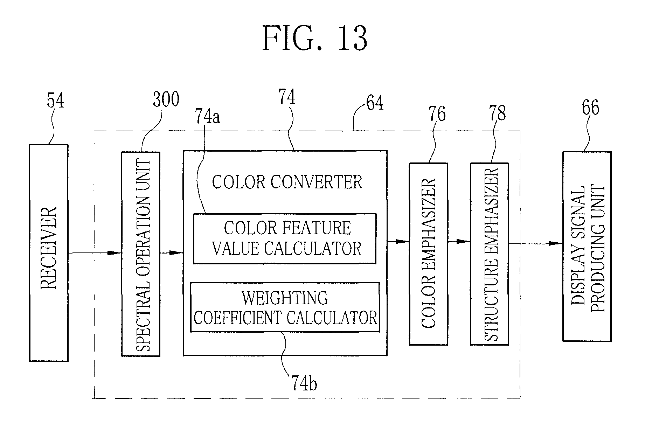

According to the third embodiment, in the special observation mode of the simultaneous type endoscope system 10, the white light, being the broad-band light (the white light obtained by the phosphor 44, the broad-band light emitted from the broad-band light source such as the xenon lamp, or the like) is applied instead of the special light. Then, as shown in Fig. 13, a spectral operation unit 300 provided between the receiver 54 and the color emphasizer 76 applies spectral operation processing to the RGB image signals, which are obtained by imaging under irradiation with the white light. The spectral operation unit 300 produces the Bn image signal having information at a wavelength of 415 nm and the Gn image signal having information at a wavelength of 540 nm. Note that, a method described in Japanese Patent Laid-Open Publication No. 2003-093336 is usable as a method for the spectral operation.

The special light image processor 64 makes a matrix conversion of the Bn image signal and the Gn image signal (denoted as Bn and Gn) based on the above expression (4) and outputs the RGB display image signals (denoted as $B_0$, Go, $R_0$), just as with the second embodiment. The color feature value x, which is used in calculation of the weighting coefficients w1 and w2, is calculated from the expression (3) based on the Bn image signal, the Gn image signal, and the R image signal, just as with the first embodiment (however, the Bn image signal is substituted for Bi of the expression (3), the Gn image signal is substituted for Gi of the expression (3), and the R image signal is substituted for Ri of the expression (3)). The special light image is produced from the RGB display image signals in the same way as the first embodiment.

Note that, according to the above embodiments, the special light observation is carried out while the endoscope is inserted in the body cavity. However, the present invention is not limited to this, the special light image may be produced after the completion of endoscopy with the use of endoscopic images recorded to a storage of the endoscope system. The special light image may be produced from capsule endoscopic images captured by a capsule endoscope.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

**Claims**

1. A processor device (16) for applying image processing to a color image signal obtained by an endoscope (12) to convert said color image signal into blue, green, and red display signals, and supplying said blue, green, and red display signals to a display means (18) for displaying an image of an observation object, said processor device (16) comprising:

   an image signal input means (54) for capturing, out of said color image signal, a blue image signal containing a blue narrow-band signal corresponding to a wavelength band in a blue region and a green image signal corresponding to a wavelength band in a green region;
   a color conversion means (74) for assigning said blue image signal as a blue display image signal, and assigning a sum of weighted said green image signal and weighted said blue image signal as a green display image signal, and assigning said green image signal as a red display image signal; and
   a display signal producing means (66) for converting said blue display image signal, said green display image signal, and said red display image signal into said blue, green, and red display signals, respectively.

2. The processor device (16) according to claim 1, wherein said color conversion means (74) includes:

   a color feature value calculator (74a) for calculating a color feature value of said observation obj ect based on said color image signal; and
   a weighting coefficient calculator (74b) for calculating, in accordance with said color feature value, a first weighting coefficient for weighting said green image signal and a second weighting coefficient for weighting said blue image signal.

3. The processor device (16) according to claim 2, wherein said color feature value increases with getting near a color of a first object (80) including at least one of residuum, residual fluid, bile, and intestinal juice present on said observation object, while said color feature value decreases with getting near a color of a second object including a brownish area; and
   with increase in said color feature value, said first weighting coefficient for weighting said green image signal is increased, and said second weighting coefficient for weighting said blue image signal is decreased.

4. The processor device (16) according to claim 3, wherein a sum of said first weighting coefficient and said second weighting coefficient is "1";
   in a case where said color feature value is equal to or less than a first threshold value, said first weighting coefficient is set at "0", and said second weighting coefficient is set at "1",
   in a case where said color feature value is equal to or more than a second threshold value, being larger than said first threshold value, said first weighting coefficient is set at "1", and said second weighting coefficient is set at "0"; and
   in a case where said color feature value is between said first threshold value and said second threshold value, said first weighting coefficient is increased and said second weighting coefficient is decreased with increase in said color feature value.

5. The processor device (16) according to one of claims 2 to 4, wherein said color feature value is calculated based on first color information that is obtained by an operation of said blue image signal and said green image signal.

6. The processor device (16) according to claim 5, wherein said image signal input means (54) captures a red image signal corresponding to a wavelength band in a red region, in addition to said blue image signal and said green image signal;
   said color feature value is calculated based on not only said first color information but also second color information that is obtained by an operation of said green image signal and said red image signal.

7. The processor device (16) according to claim 6, wherein said color feature value is a sum of weighted said first color information and weighted said second color information, and said first color information is more heavily weighted than said second color information.

8. The processor device (16) according to one of claims 1 to 7, wherein a first object (80) including at least one of residuum, residual fluid, bile, and intestinal juice present on said observation object is displayed in a yellowish color on said display means (18).

9. The processor device (16) according to claim 8, wherein structure (82) under said first object (80) is displayed translucently on said display means (18).

10. An endoscope system (10) comprising:

A. an endoscope (12) for taking an image of an observation object and outputting a color image signal;
B. a processor device (16) including:

an image signal input means (54) for capturing, out of said color image signal, a blue image signal containing a blue narrow-band signal corresponding to a wavelength band in a blue region and a green image signal corresponding to a wavelength band in a green region;
a color conversion means (74) for assigning said blue image signal as a blue display image signal, and assigning a sum of weighted said green image signal and weighted said blue image signal as a green display image signal, and assigning said green image signal as a red display image signal; and
a display signal producing means (66) for converting said blue display image signal, said green display image signal, and said red display image signal into said blue, green, and red display signals, respectively; and

C. a display means (18) for displaying said image of said observation object based on said blue, green, and red display signals.

11. The endoscope system (10) according to claim 10, wherein said endoscope (12) includes:

a first lighting means (14, 41, 24a) for applying illumination light containing blue narrow-band light to said observation object; and
a color image pickup device (48) for taking said image of said observation object and outputting said blue image signal and said green image signal.

12. The endoscope system (10) according to claim 10 or 11, wherein said endoscope (12) includes:

a second lighting means (14, 41, 24a) for alternately applying blue narrow-band light and green narrow-band light to said observation object; and
a monochrome image pickup device (206) for taking said image of said observation object, and outputting said blue image signal containing said blue narrow-band signal corresponding to said blue narrow-band light, and outputting said green image signal containing a green narrow-band signal corresponding to said green narrow-band light.

13. The endoscope system (10) according to one of claims 10 to 12, wherein said endoscope (12) includes:

a third lighting means (14, 41, 24a) for applying broad-band light in a wavelength band extending from a blue region to a red region to said observation object;
a color image pickup device (48) for taking said image of said observation object and outputting said color image signal; and
a spectral operation unit (300) for producing said blue image signal and said green image signal by a spectral operation of said color image signal.

14. An operation method of an endoscope system (10) comprising the steps of:

actuating an image signal input means (54) and capturing, out of a color image signal obtained by an endoscope (12), a blue image signal containing a blue narrow-band signal corresponding to a wavelength band in a blue region and a green image signal corresponding to a wavelength band in a green region;
actuating a color conversion means (74) and assigning said blue image signal as a blue display image signal, and assigning a sum of weighted said green image signal and weighted said blue image signal as a green display image signal, and assigning said green image signal as a red display image signal;

actuating a display signal producing means (66) and converting said blue display image signal, said green display image signal, and said red display image signal into blue, green, and red display signals, respectively; and displaying an image of an observation object on a display means (18) based on said blue, green, and red display signals.

# FIG. 1

EP 2 803 313 A1

# FIG. 2

EP 2 803 313 A1

# FIG. 3A

LIGHT
INTENSITY

BLUE LASER BEAM ⎫
FLUORESCENCE ⎭ WHITE LIGHT

445         700    WAVELENGTH
(nm)

# FIG. 3B

LIGHT
INTENSITY

VIOLET LASER BEAM ⎫
BLUE LASER BEAM ⎬ SPECIAL LIGHT
FLUORESCENCE ⎭

405   445        700   WAVELENGTH
(nm)

# FIG. 4A

# FIG. 4B

# FIG. 5A

# FIG. 5B

# FIG. 6

# FIG. 7

# FIG. 8

START

SPECIAL OBSERVATION MODE

CAPTURE RGB IMAGE SIGNALS

CALCULATE COLOR FEATURE VALUE x

CALCULATE WEIGHTING COEFFICIENTS w1, w2

MATRIX CONVERSION
$(Ro,Go,Bo) = (Gi, w1 \times Gi + w2 \times Bi, Bi)$

DISPLAY SPECIAL LIGHT IMAGE

END

# FIG. 9

# FIG. 10

ZOOMING OPERATION UNIT — 22c

MODE SWITCH — 22b

12 — 16 — 200

IMAGING LENS — 46
ZOOMING LENS — 47
24a — 24b
IMAGE SENSOR — 206
CDS/AGC — 50
A/D — 52
DSP — 56
NOISE REMOVER — 58
54
60
COLOR CONVERTER — 68
COLOR EMPHASIZER — 70
STRUCTURE EMPHASIZER — 72
62
DISPLAY SIGNAL PRODUCING UNIT — 66
MONITOR — 18

LIGHTING LENS — 45
LG — 41
FILTER SWITCHER — 205
ROTARY FILTER — 204
BROAD-BAND LIGHT SOURCE — 202
14

COLOR CONVERTER — 74
COLOR FEATURE VALUE CALCULATOR — 74a
WEIGHTING COEFFICIENT CALCULATOR — 74b
COLOR EMPHASIZER — 76
STRUCTURE EMPHASIZER — 78
64

EP 2 803 313 A1

# FIG. 11

# FIG. 12

# FIG. 13

RECEIVER 54

SPECTRAL OPERATION UNIT 300

COLOR CONVERTER 74

COLOR FEATURE VALUE CALCULATOR 74a

WEIGHTING COEFFICIENT CALCULATOR 74b

64

COLOR EMPHASIZER 76

STRUCTURE EMPHASIZER 78

DISPLAY SIGNAL PRODUCING UNIT 66

Europäisches
Patentamt
European
Patent Office
Office européen
des brevets

**EUROPEAN SEARCH REPORT**

Application Number

EP 14 16 7618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 481 342 A1 (FUJIFILM CORP [JP]) 1 August 2012 (2012-08-01) * paragraph [0126] - paragraph [0152] * ----- | 1,10-12, 14 | INV. A61B1/00 A61B1/06 A61B1/04 |
| A | US 2001/007921 A1 (HAYASHI KATSUMI [JP]) 12 July 2001 (2001-07-12) * paragraphs [0055] - [0081] * ----- | 1-14 | |
| A | WO 2013/042396 A1 (OLYMPUS MEDICAL SYSTEMS CORP [JP]; YAMAZAKI KENJI [JP]; IGARASHI MAKOT) 28 March 2013 (2013-03-28) * paragraphs [0023] - [0057] * | 1-14 | |
| A,P | & US 2013/172675 A1 (YAMAZAKI KENJI [JP] ET AL) 4 July 2013 (2013-07-04) * paragraphs [0023] - [0057] * ----- | 1-14 | |

TECHNICAL FIELDS
SEARCHED        (IPC)

A61B
G09G

The present search report has been drawn up for all claims

| Place of search | Date of completion of the search | Examiner |
|---|---|---|
| Munich | 5 September 2014 | Rivera Pons, Carlos |

EPO FORM 1503 03.82 (P04C01)

## ANNEX TO THE EUROPEAN SEARCH REPORT
## ON EUROPEAN PATENT APPLICATION NO.

EP 14 16 7618

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2014

| Patent document cited in search report | | Publication date | Patent family member(s) | | Publication date |
|---|---|---|---|---|---|
| EP 2481342 | A1 | 01-08-2012 | EP 2481342 A1 | | 01-08-2012 |
| | | | JP 5554253 B2 | | 23-07-2014 |
| | | | JP 2012152414 A | | 16-08-2012 |
| | | | US 2012197077 A1 | | 02-08-2012 |
| US 2001007921 | A1 | 12-07-2001 | JP 2001178672 A | | 03-07-2001 |
| | | | US 2001007921 A1 | | 12-07-2001 |
| WO 2013042396 | A1 | 28-03-2013 | CN 103533878 A | | 22-01-2014 |
| | | | EP 2692276 A1 | | 05-02-2014 |
| | | | JP 5363680 B2 | | 11-12-2013 |
| | | | US 2013172675 A1 | | 04-07-2013 |
| | | | WO 2013042396 A1 | | 28-03-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030176768 A
- US 20080294105 A
- US 20080281154 A
- US 20090036741 A
- JP 2003093336 A